Europäisches Patentamt

(19) European Patent Office

Office européen des brevets

(11) Publication number: **0 087 404**
**A2**

(12) **EUROPEAN PATENT APPLICATION**

(21) Application number: **83850040.3**

(22) Date of filing: **16.02.83**

(51) Int. Cl.³: **C 12 P 19/08**

(30) Priority: **18.02.82 SE 8201021**

(43) Date of publication of application:
**31.08.83 Bulletin 83/35**

(84) Designated Contracting States:
**CH DE FR GB IT LI NL SE**

(71) Applicant: **AB SORIGONA**
**Box 134**
**S-245 00 Staffanstorp(SE)**

(72) Inventor: **Jarl, Kurt**
**Norrevangsgatan 11**
**S-232 00 Arlöv(SE)**

(72) Inventor: **Engblom, Curt**
**Tvättmästarvägen 27**
**S-232 02 Akarp(SE)**

(74) Representative: **Burman, Tore et al,**
**Bergling & Sundbergh AB P.O. Box 7645**
**S-103 94 Stockholm(SE)**

(54) A process for the production of dextran.

(57) A process for preparing by means of microorganisms or enzymes, dextrane having a molecular weight suitable for clinical use starting from a saccharose-containing substrate, wherein it is carried out continuously with continuous supply of substrate and microorganisms or enzymes and continuous discharge of dextran-containing reaction mixture from which the dextran is then recovered.

EP 0 087 404 A2

TITLE OF INVENTION:

A process for the production of dextran.

The present invention relates to a process for producing dextran using microorganisms or enzymes, the dextrane produced having a molecular weight suitable for clinical use.

Dextran, which is a high molecular substance built up from glucose units with α-1,6-glycoside linkages, is predominantly used as synthetic blood plasma. Dextran is manufactured under the influence of bacteria, mostly of the genus Leuconostoc, which have the ability of synthesizing dextran out of saccharose. The molecular weight suitable for medicinal purposes often lies below about 300000 and particularly within the range from 30000 to 100000.

In batch-wise manufacture of dextran using bacteria or enzymes there will be, however, obtained a dextran of a molecular weight which is essentially higher, of the order of several millions. For clinical use of the dextran its molecular weight must be reduced to the desired range and this generally takes place by acid hydrolysis and fractionation of the dextran. This post treatment of the dextran raw material is associated with problems and costs. Thus, the acid hydrolysis is difficult to control so that the main part of the hydrolysis product lies within the desired range. In practically applied hydrolysis fractionation must therefore be resorted to for obtaining a clinically acceptable product and the yield losses are of significance.

Thus, the object of the present invention is to provide a new process for preparing, by fermentation or enzymatically, dextran of a lower molecular weight suitable for clinical use.

Another object of the invention is to provide a process enabling manufacture of a dextran which has a molecular weight suitable for clinical use without having to resort to hydrolysis of the product for obtaining the desired molecular weight.

In accordance with the present invention it has surprisingly been found that if the fermentative and/or enzymatic process is performed continuously under continuous supply of substrate and microorganisms or enzymes and continuous discharge of the dextran-containing reaction mixture there will be obtained a dextran which has a molecular weight suitable for clinical use. Thus, it has been found that in continuous operation the dextran obtained for the predominant part has a molecular weight below about 300000 and often below about 100000.

It is preferred to produce the dextran by fermentation, the reaction mixture discharged being subjected to cell separation. In connection with the process of the present invention this separation of cells is in practical operation easier to accomplish than is the case in conventional dextran manufacture in view of the fact that the reaction mixture obtained has a lower viscosity enabling easier separation of the cells. After cell separation the dextran is recovered from the reaction mixture by precipitation, for example by adding ethanol. Since the solubility of the dextran in ethanol is strongly dependent on the molecular weight also molecular weight fractionation of the dextran by using ethanol can be accomplished which per se constitutes conventional technique.

In other respects the process of the invention is performed in a traditional manner and any dextran--producing organism can be utilized in the process. However, it is preferred to use an organism of the genus Leuconostoc, particularly the species Leuconostoc mesenteroides. In the practical embodiment presented below the strain Leuconostoc mecenteroides NRRL B512 has been used but the invention is, of course, by no means limited to the use of just this strain.

In connection with the process according to the present invention the enzyme production or cell cultivation can be performed batchwise or continuously in accordance with conventional techniques. The substrate for the enzyme production contains saccharose, suitably in a quantity within the range from 10 to 50 g/liter, and yeast extract and other nitrogen substances and growth factors in accordance with conventional art. Inorganic salts cover the requirements of magnesium, phosphorus, sulphur, trace elements etc. and can be varied in regard to components and concentrations.

Sterilisation can take place in the usual manner, for example by sterile filtration or in another way. The enzyme production is suitably performed within the temperature range 10 to 50°C, preferably 23 to 30°C. The cultivation is suitably carried out without or with only slight aeration.

The cell cultivation is suitably carried out within the pH-range 5.5 to 8.0, particularly 6.2 to 7.0. In batch-wise cultivation without pH-control the cultivation will be initiated at a pH of about 6.8, the pH spontaneously falling to about 5.0 at the termination of growth. If the culture or enzyme solution is to be stored the pH should be lowered to about 5.0 and the temperature should be maintained at about 4°C in order to avoid losses of activity.

When using continuous cell cultivation the dilution rate, i.e. the ratio between ingoing flow and liquid volume in the reaction vessel, may be varied between 0.05 and 0.7 $h^{-1}$, preferably $\geq 0.1\ h^{-1}$, high enzyme activities being obtainable.

Dextran synthesis performed in accordance with the invention takes place continuously with continuous supply of substrate and enzyme solution (with or without cells) to a volume of liquid in the reaction vessel, said volume being maintained constant by discharge of dextrane-containing reaction mixture. The synthesis is suitably carried out in a reaction vessel equipped with stirrer and with means for temperature and pH-control.

The saccharose concentration of the substrate can vary freely up to about 70 per cent by weight. The saccharose concentration for the mixture entering the reaction vessel, i.e. the proportion of ingoing substrate flow to total input flow times the saccharose concentration of the substrate, should, however, preferably be adjusted to about 10 to 25 %, so that the corresponding dextran concentration will be suitable for the recovery later on.

Sterilization of the substrate takes place according to known technique and may optionally be supplemented with or replaced by a sterile protection in the reaction vessel, for example by addition of toluene which does not damage the enzyme.

The dextran synthesis is operated at a temperature suitably within the range from 10 to $50^{o}$C, preferably from 15 to $23^{o}$C. The pH of the reaction zone is suitably adjusted within the range 4.0 to 8.0, preferably 4.8 to 6.0.

The dilution rate, i.e. the proportion of total input flow to liquid volume in the reaction vessel, is adjusted to the enzyme activity and the saccharose concentration of ingoing mixture so that the major part of the saccharose, i.e. 80 to 95 %, will be converted into dextran in the leaving reaction mixture.

By selecting such parameters as dilution rate, temperature, saccharose concentration in the input

mixture etc., in a suitable manner the high-molecular fraction can be substantially reduced and the molecular weight distribution of the dextran produced optimized in other respect.

As previously mentioned there is obtained by the fact that the viscosity of the reaction mixture from the reaction vessel is relatively low, the advantage that bacterial cells can be separated by filtration or centrifugation. This is as indicated a further advantage compared to conventional batch-wise dextran fermentation where the high viscosity of the product makes such separation impossible.

After the cell separation the dextrane can be recovered in a conventional manner, for example by fractionated precipitation with ethanol, methanol or the like. Fractions in the desired molecular weight ranges are precipitated a number of times, the solvent is separated and after optional further purification the dextran may be spraydried if desired. A low content of high molecular dextran fraction optionally present may be hydrolyzed in a conventional manner if the quantity thereby obtained is sufficient to make it economically interesting. However, the technique of this invention enables manufacture of dextran having a molecular weight, which to the predominant part is suitable for clinical use.

In connection with the starting up of processes when applying the process of the present invention a steady state condition will be reached in a shorter period of time if the reaction vessel is charged in advance with the right dextran volume (of appropriate molecular weight), substrate and cell culture or enzyme solution to the volume and in the proportions corresponding to the conditions at continuous operation. The invention will in the following be described by non-limiting examples in connection to the appended drawing, wherein:

Fig. 1 shows a diagram on the quantity of precipitated dextran as a function of the ethanol concentration;

Fig. 2 shows a diagram on eluation of dextran from Sepharos 4 B showing the quantity of dextran eluated as a function of the eluation volume; and

Fig. 3 shows the molecular weight distribution of dextran prepared in accordance with the invention and of clinical dextran found on the market.

In the examples the percentages given relate to weight if not otherwise stated.

EXAMPLE 1

Enzyme production

| Substrate: | Saccharose | 20 g/l |
| | Yeast extract | 2 g/l |
| | $KH_2PO_4$ | 1 g/l |
| | $MgSO_4 \cdot 7H_2O$ | 0.2 g/l |
| | NaCl | 0.01 g/l |
| | $FeSO_4 \cdot 7H_2O$ | 0.01 g/l |
| | $MnSO_4 \cdot H_2O$ | 0.01 g/l |

pH adjusted to 6.7-6.8.

The substrate components are dissolved in de-ionized water in the above concentrations and are autoclaved at 121°C for 30 minutes. After inoculation with 100 ml inoculate culture of Leuconostoc mesenteroides B512 grown on a substrate of the same composition incubation was carried out in a 2 liter fermentor (volume of liquid = 1 liter) while stirring and controlling pH. The pH was held at 6.7 and the temperature at 25°C.

After batch-wise cultivation for 24 hours, continuous substrate input = 95 ml/h was initiated and an equally large output flow from the fermentor (constant volume of liquid in the fermentor). After about 24 hours there was obtained in the centrifuged cell-free solution a dextran sucrase activity of about 25 DSU-units (1 DSU-unit = the quantity of dextran sucrase producing 1 mg of fructose (reducing sugar) from saccharose in an hour at $23^{\circ}C$, pH = 5.2) which could be then maintained for more than 10 days.

The dextran synthesis was carried out in an 8-liter fermentation vessel with stirrer and pH-control (pH = 5.2) and continuous input flows of the above enzyme solution (125 ml/h) and sterile 22 % saccharose solution (250 ml/h). The temperature was held at $23^{\circ}C$. The volume of liquid in the vessel was kept constant at 4 liters by means of an overflow.

. Analysis of the output flow showed at steady state conditions a dextran content corresponding to 80-90 % yield of the theoretically calculated yield (1 g saccharose gives theoretically 0.47 g dextran). The resulting solution had a relatively low viscosity (as compared to that obtained in batch-wise fermentation according to Example 2), and the dextran shows after purification from fructose etc. by repeated precipitations with ethanol an intrinsic viscosity $\eta$ = 0.40. This may be compared with $\eta$ = 1.1 which is normally obtained for dextran prepared by conventional batch-wise procedure (see Example 2, molecular weight $>10^{6}$) and $\eta$ = 0.21, 0.26, 0.31, 0.36, 0.42, 0.52 for dextran preparations obtained after acid-hydrolysis and careful fractionation (Pharmacia-products T40, T70, 110, T150, T250, T500) having average molecular weights of $M_{w}$ = 41 800, 67 600, 100 500, 147 000, 243 000, 510 000 respectively.

The continuous process according to the invention thus results in a considerably lower molecular weight than the batch-wise process and largely corresponding to the range relevant to the clinical use.

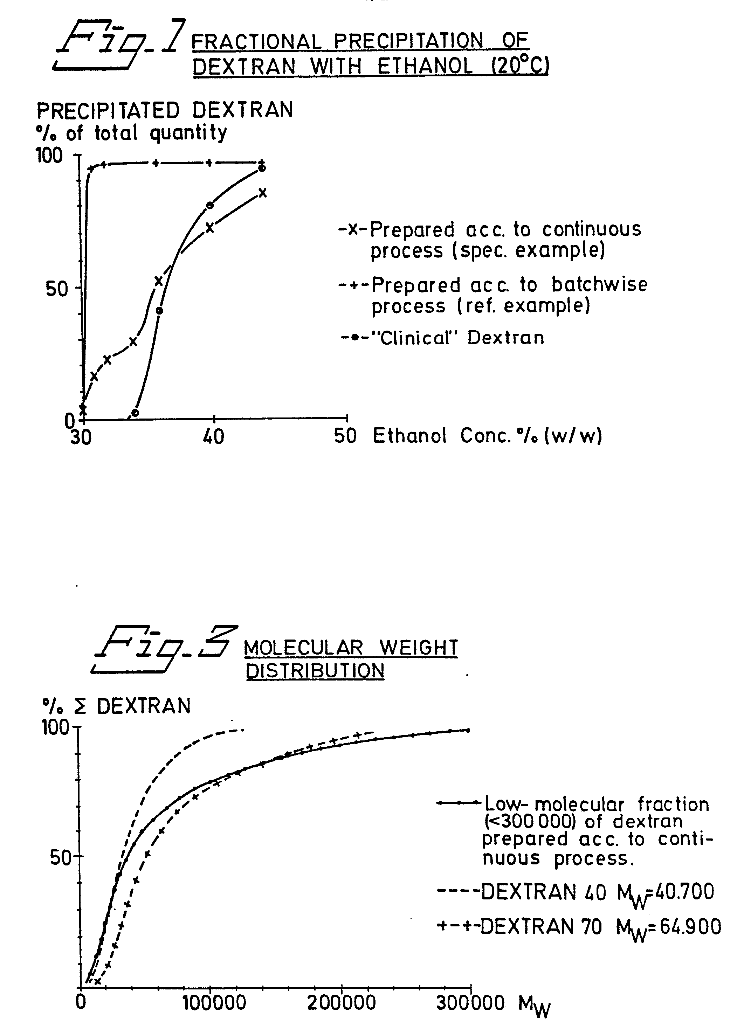

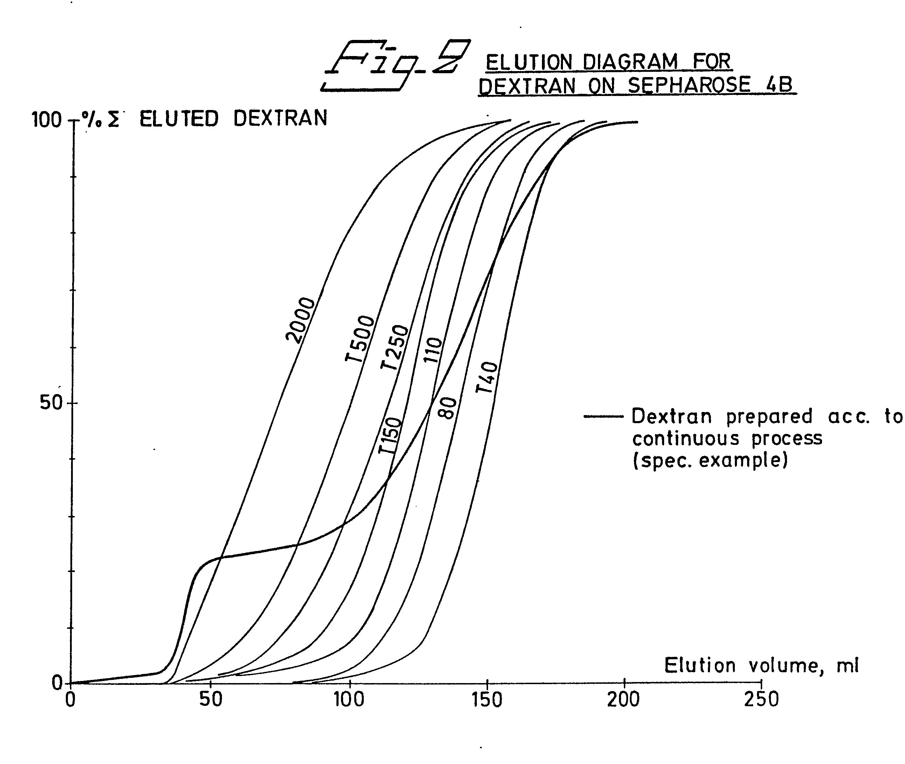

Further investigation by analytical fractionated precipitation with ethanol (Fig. 1) and gel filtration (Fig. 2) on a Sepharos 4B column reveals a bimodal molecular weight distribution with a minor fraction in the high-molecular area ($M_w > 10^6$) and the predominant part in the area of current interest to the clinical use.

The low molecular fraction (molecular weight $< 300\ 000$) in fact shows a molecular weight distribution which closely corresponds to those of the clinical preparations dextran 40 and dextran 70 (Fig. 3).

EXAMPLE 2: BATCH-WISE DEXTRAN FERMENTATION (REFERENCE)

Substrate:   Saccharose          120 g/l
             Yeast extract       0.5 g/l
             $KH_2PO_4$          0.5 g/l
             pH adjusted to 6.8-7.0.

1.5 liters of substrate in a 2-liter flask were autoclaved at 121°C for 20 minutes. It was inoculated with 30 ml of an inoculate culture (Leuconostoc mesenteroides B512) cultivated at 23°C, 36 hours on the same substrate supplemented with peptone 1 g/l, $MgSO_4 \cdot 7H_2O$ 0.2 g/l.

After 48 hours of incubation at 23°C at stationary conditions without aeration pH had decreased to 4.8 and a highly viscous solution was obtained. Analysis showed a dextran content corresponding to 88 % of that theoretically calculated. The dextran was of high molecular type and the molecular weight was $> 10^6$.

It should be observed that the invention is by no means restricted to the examples described above which merely are intended to illustrate the practical application of the invention. Thus, other dextran producing microorganisms may be used, the composition of the substrates may be varied and the experimental parameters in other respects may be modified.

PATENT CLAIMS.

1.   A process for preparing by means of microorganisms or enzymes dextran having a molecular weight
suitable for clinical use starting from a saccharose-containing substrate, characterized thereby that it is
carried out continuously with continuous supply of
substrate and microorganisms or enzymes and continuous
discharge of dextran—containing reaction mixture from
which the dextran is then recovered.

2.   A process according to claim 1, characterized
thereby that it is carried out in a fermentative manner
using a dextran—producing species of Leuconostoc.

3.   A process according to claim 2, characterized
thereby that the species mesenteroides is used.

4.   A process according to claim 3, characterized
by using the strain Leuconostoc mesenteroides NRRL B512.

5.   A process according to any preceding claim
for preparing dextran by fermentation, characterized
thereby that the reaction mixture discharged is subjected to cell-separation and then dextran precipitation.

6.   A process according to any preceding claim,
characterized thereby that cultivation of microorganisms
for the enzyme production is performed continuously and in
parallel with the dextran manufacture.

7.   A process according to any preceding claim,
characterized thereby that it is performed at a
temperature within the range of about 10-50°C.

8.   A process according to any preceding claim
for the manufacture of dextran the predominant part of
which has a molecular weight lower than about 300 000.

9.   A process according to any preceding claim,
characterized thereby that in order to obtain a steady state condition in a shorter period of time in connection with starting up the process dextran of the desired molecular weight is added to the reaction zone and also other components to the desired volume and in the desired proportions.

10.  Dextran produced by the process according to any preceding claim.

0087404

1/2

**Fig. 1** FRACTIONAL PRECIPITATION OF DEXTRAN WITH ETHANOL (20°C)

PRECIPITATED DEXTRAN
% of total quantity

-x-Prepared acc. to continuous process (spec. example)

-+-Prepared acc. to batchwise process (ref. example)

-•-"Clinical" Dextran

**Fig. 3** MOLECULAR WEIGHT DISTRIBUTION

% Σ DEXTRAN

Low-molecular fraction (<300 000) of dextran prepared acc. to continuous process.

----DEXTRAN 40 $M_W$=40.700

+-+-DEXTRAN 70 $M_W$=64.900

*Fig. 9* ELUTION DIAGRAM FOR DEXTRAN ON SEPHAROSE 4B

100 — % Σ ELUTED DEXTRAN

2000
T500
T250
110
T150
80
T40

50

—— Dextran prepared acc. to continuous process (spec. example)

Elution volume, ml

0

0    50    100    150    200    250